# EUROPEAN PATENT APPLICATION

(11) **EP 3 686 648 A1**
(43) Date of publication of application: **29.07.2020**
(21) Application number: 18852762.6
(22) Date of filing: 19.07.2018
(51) Int. Cl.: G02B 27/00, G02B 27/01, G06N 99/00, G06F 3/01, A61B 5/16, A61B 5/0476

(54) **HEAD-MOUNTED DISPLAY DEVICE**

(30) Priority: 18.09.2017 KR 20170119795
(71) Applicant: Looxid Labs Inc., Yuseong-gu, Daejeon 34141 (KR)
(72) Inventor: CHAE, Yong Wook, Seoul 06247 (KR); LEE, Hong Gu, Seoul 06247 (KR); CHOI, Tae Jun, Seoul 06247 (KR); CHOI, Ki Bum, Seoul 06247 (KR)
(74) Representative: Fabry, Bernd
(86) International application number: PCT/KR2018/008174
(87) International publication number: WO 2019/054621

(57) **Abstract**

Disclosed herein is a head-mounted display device. The head-mounted display device comprises: a display unit disposed to face a user's face when the user wears the device and configured to provide the user with virtual reality images via an image output means disposed on one side thereof; a first biometric data acquiring unit disposed at the display unit and configured to capture the user's face accommodated in the display unit when the user wears the device to acquire first biometric data; and a second biometric data acquiring unit disposed at the display unit and configured to come in contact with the user's skin when the user wears the device to acquire second biometric data.

According to an exemplary embodiment of the present disclosure, the head-mounted display device can not only provide a user with virtual reality image but also acquire biometric data from the user's face who is watching the virtual reality image to thereby check the user's cognitive and emotional conditions. Accordingly, it is possible to develop contents including various experience elements. Furthermore, it is possible to improve satisfaction of the user's experience by providing contents suitable for the user's current conditions in real-time.

## Description

### [TECHNICAL FIELD]

The present disclosure relates to a head-mounted display device, and more particularly, to a head-mounted display device which can be easily worn and can measure biometric data of a user.

### [BACKGROUND ART]

Typically, a head-mounted display (HMD) device is worn on a user's head and provides the user with virtual reality (VR) images to allow for spatial and temporal experience similar to the reality.

Such a head-mounted display device includes a body portion implemented as a goggle that is located around a user's eyes, and a strap portion implemented as a band that is connected to the body portion and is fixedly worn around the user's head. As a means for outputting a virtual reality image, a portable terminal device such as a smart phone is mounted at the body portion or a display device such as a monitor connected to a PC or the like is disposed.

Previously, a head-mounted display device merely provides a user with visual images output from the image output means installed in a portable terminal device, such that the user can enjoy a visual experience only. As a result, existing head-mounted display devices do not have many applications, and the type of the contents provided from the image output means is very limited.

In existing head-mounted display devices, the body portion and the strap portion are formed integrally or the strap portion is implemented as a band that surrounds the head and is made of a rubber material. Accordingly, it is not easy for a user to put on the existing head-mounted display devices. In addition, when a user wants to interrupt the virtual reality experience in order to look around, the user has to take off the device from the head, which is inconvenient.

In addition, since the body portion of an existing head-mounted display device is supported by a rubber band around the user's head, it can not be stably fixed to the user's face and thus may be deviated from the user's face during the experience.

In addition, in existing head-mounted display devices, a cushioning member is integrally disposed on a surface of the body, which comes in contact with the user's face. Accordingly, if the cushioning member is deformed or broken, it was not possible to replace it separately. Therefore, if the cushioning member is deformed or broken, the user has to replace the whole head-mounted display device or has to use the head-mounted display device with the cushioning material deformed or broken.

### [SUMMARY OF INVENTION]

### [Technical Problem]

In view of the above, an object of the present disclosure is to provide a head-mounted display device that can be easily put on and can measure biometric data of a user to sense the user's physical, cognitive and emotional changes.

It should be noted that objects of the present invention are not limited to the above-mentioned object; and other objects of the present invention will be apparent to those skilled in the art from the following descriptions.

### [Technical Solutions]

According to an aspect of the present disclosure, there is provided a head-mounted display device, comprising: a display unit disposed to face a user's face when the user wears the device and configured to provide the user with virtual reality images via an image output means disposed on one side thereof; a first biometric data acquiring unit disposed at the display unit and configured to capture the user's face accommodated in the display unit when the user wears the device to acquire first biometric data; and a second biometric data acquiring unit disposed at the display unit and configured to come in contact with the user's skin when the user wears the device to acquire second biometric data.

The display assembly may include: a strap portion that is wearable on the user's head; and a display body that is rotatably coupled to the strap portion and being positioned in front of the user's face as the user operates, wherein the display body has a face-accommodating space on one side thereof where the user's face is to be accommodated, and a display-accommodating space on an opposite side thereof where the image output means is to be accommodated.

The strap portion may include: a strap body comprising a band portion having a head-accommodating space formed therein for receiving the user's head and fitting around the user's head when the user wears the device, and a band support extended from the band portion and rotatably coupled to a rotation support portion; an inner-diameter adjusting means disposed at the strap body to allow the user to adjust an inner diameter of the strap body; and a buffer portion disposed on an inner surface of the band portion to support the user's head when the user wears the device.

The inner-diameter adjusting means may include: an adjustment portion comprising a support plate coupled to the band portion, and a rotation adjusting member rotatably disposed at a center of the support plate and having a pinion gear on one side thereof; a guide plate disposed on an inner surface of the band portion to face the adjustment portion and has a guide rail formed on one surface thereof in a longitudinal direction; and a moving member that is accommodated in the guide rail and has a rack gear on one side thereof to be engaged with the pinion gear, and a coupling means coupled to the band portion on an opposite side thereof, wherein the moving member moves toward one side or an opposite side when the rotation adjusting member is rotated to adjust the inner diameter of the band portion.

The display body may include: a housing having openings in one side and the other side thereof which penetrate the housing; a rotation support portion accommodated in the housing to be rotatably coupled to the wearable portion and restricting an rotation angle of the display body; a clip frame disposed at the opening on the one side of the housing, having the display accommodating space on one side thereof, and linearly movable along the penetration direction of the housing; a face accommodating frame disposed at the opening on the other side of the housing and having the face accommodating space therein; lenses disposed at the face accommodating frame; and a focusing means coupled to the clip frame and configured to move the clip frame according to the user's operation to separate the display accommodating space from the lenses by a predetermined display.

The rotation support member may include: a rotation support body disposed at the display body and capable of accommodating a part of the strap portion; a rotation shaft penetrating the rotation support body and a part of the strap portion accommodated in the rotation support body to rotatably connect the rotation support body with the strap portion; a rotation restricting member coupled to the rotation support body and having projections on a surface thereof that faces the part of the strap portion so that the part of the strap portion is held by the projections when the display body is rotated; and an elastic member connecting the rotation support body with the part of the strap portion and elastically support the rotation support body.

The clip frame may include: a shielding portion accommodated in the housing portion to be disposed in front of the face-accommodating frame and comprising a hood surrounding the lenses and an extended portion extended from an end of the hood outwardly to be supported on an inner surface of the housing portion; a display support portion disposed in front of the shielding portion and comprising a first support portion for supporting a vertical load applied to the image output means, and a second support portion extended upwardly from an end of the first support portion for supporting the image output means supported by the first support portion toward the lenses; a cushion pad disposed at the first support portion and the second support portion to be in contact with the image output means; and a linearly-moving member disposed at the shielding portion, having a rack gear formed on a surface thereof in a longitudinal direction to be coupled with the focusing means, and converting a rotation movement of the focusing means into a linear movement to move the shielding portion linearly.

The face-accommodating frame may include a face-accommodating body having a coupling means to be coupled with the second biometric data acquiring unit on one surface thereof and having the face-accommodating space where the user's eyes and nose are to be accommodated; a shielding pad disposed at the face-accommodating body and in tight contact with the user's nose when the user wears the device to block light from being introduced into the face-accommodating space; and a socket disposed at the face-accommodating body and electrically connected to the second biometric data acquiring unit.

The focusing means may include: a pinion gear engaged with the rack gear of the linearly-moving member; a power transmission gear for transmitting rotational power to the pinion gear; and a handle coupled to the power transmission gear, partially exposed out of the housing portion, and transmitting rotational power to the power transmission gear according to the user's operation.

The first biometric data acquiring unit may include: an IR filter disposed in the face-accommodating frame and inclined with respect to a center axis of the lenses at a predetermined angle; an angle adjusting unit disposed between the clip frame and the face-accommodating frame and ratable within a predetermined angle; a capturing unit disposed at the angle adjusting unit and capable of capturing at least a part of the user's face to acquire the first biometric data; and an IR light source disposed at the angle adjusting unit and disposed around the capturing unit to emit light.

The angle adjusting unit may include: a frame disposed in the housing portion and having an accommodating space therein; an operating part comprising a main shaft rotatably coupled to the frame, a main gear coupled to the main shaft and rotatable with the main shaft, and an operating means coupled to the main shaft to rotate with the main gear and the main shaft, wherein a part of the operating means is exposed out of the housing portion so that the user can operate with it; and a rotating body comprising a rotating body accommodated in the frame, wherein the capturing unit and the IR light source are disposed on an inner side thereof, a driven shaft coupled to the rotating body and rotatably coupled to the frame, and a driven gear coupled with one side of the driven shaft to be engaged with the main gear and rotated with the rotating body and the driven shaft as the main gear is rotated.

The second biometric data acquiring unit may include: a mask body having a coupling means on one surface thereof that faces the display unit, and being detachably attached to the display unit; a sensing means disposed at the mask body and coming in contact with the user's skin when the user wear the device to sensing a biometric signal from the user to acquire the second biometric data; and a connector disposed at the mask body and electrically connected to the sensing means, wherein the connector is connected to the socket disposed at the display unit to transmit the second biometric data sensed by the sensing means.

### [Advantageous Effects]

According to an exemplary embodiment of the present disclosure, the head-mounted display device can not only provide a user with virtual reality image but also acquire biometric data from the user's face who is watching the virtual reality image to thereby check the user's cognitive and emotional conditions. Accordingly, it is possible to develop contents including various experience elements. Furthermore, it is possible to improve satisfaction of the user's experience by providing contents suitable for the user's current conditions in real-time.

Further, since the display body is rotatably coupled to the strap portion and the strap portion is in the form of an annular structure which can be put on by one hand, the user can easily put it on and take it off to look around as desired. As a result, the convenience can be improved.

Further, the display body can be stably fixed to the user's face by the annular strap portion in the form of the structure. Furthermore, even if the user takes a strenuous action during the experience, it is possible to prevent the display body from being deviated from the user's face.

Further, as the mask body supporting the user's face can be detached from the display body, it is possible to easily replace the mask body, thereby increasing the lifetime of the device while saving the cost.

### [BRIEF DESCRIPTION OF DRAWINGS]

FIG. 1 is a perspective view of a front structure of a head-mounted display device according to an exemplary embodiment of the present disclosure;
FIG. 2 is a perspective view of a rear structure of the head-mounted display device according to the exemplary embodiment of the present disclosure;
FIG. 3 is a front view of the head-mounted display device according to the exemplary embodiment of the present disclosure;
FIG. 4 is a cross-sectional view taken along line IV - IV of FIG. 3;
FIG. 5 is a side view of the head-mounted display device according to the exemplary embodiment of the present disclosure;
FIG. 6 is a cross-sectional view taken along line VI - VI of FIG. 5;
FIG. 7 is a rear view of a display body of the head-mounted display device according to the exemplary embodiment of the present disclosure after a face-accommodating frame is removed from it;
FIG. 8 is a cross-sectional view taken along line VIII - VIII of FIG. 3;
FIG. 9 is a cross-sectional view taken along line IX - IX of FIG. 3;
FIG. 10 is an enlarged view of portion A of FIG. 9;
FIG. 11 is a plan view of the display body of the head-mounted display device according to the exemplary embodiment of the present disclosure after a housing portion is removed from it;
FIG. 12 is a rear view of the display body of the head-mounted display device according to the exemplary embodiment of the present disclosure;
FIG. 13 is a front view of a first biometric data acquiring unit of the head-mounted display device according to the exemplary embodiment of the present disclosure;
FIG. 14 is a perspective view of a rear structure of the first biometric data acquiring unit of the head-mounted display device according to the exemplary embodiment of the present disclosure;
FIG. 15 is a front view of a frame of a second biometric data acquiring unit of the head-mounted display device according to the exemplary embodiment of the present disclosure;
FIG. 16 is a longitudinal sectional view of the second biometric data acquiring unit of the head-mounted display device according to the exemplary embodiment of the present disclosure;
FIG. 17 is a view showing a way how the second biometric data acquiring unit of the head-mounted display device according to the exemplary embodiment of the present disclosure comes in contact with the user;
FIG. 18 is a front view of a buffer portion disposed at frame of the second first biometric data acquiring unit of the head-mounted display device according to the exemplary embodiment of the present disclosure;
FIG. 19 is a cross-sectional view of a modification of a sensing means at the second biometric data acquiring unit of the head-mounted display device according to the exemplary embodiment of the present disclosure;
FIG. 20 is a front view of the second biometric data acquiring unit of the head-mounted display device according to the exemplary embodiment of the present disclosure.
FIG. 21 is a view showing a modification of a sensing means disposed at the second biometric data acquiring unit of the head-mounted display device according to the exemplary embodiment of the present disclosure;
FIG. 22 is a cross-sectional view of a connector at the second biometric data acquiring unit of the head-mounted display device according to the exemplary embodiment of the present disclosure;
FIG. 23 is a rear view of the second biometric data acquiring unit of the head-mounted display device according to the exemplary embodiment of the present disclosure.

### [DESCRIPTION OF EMBODIMENTS]

Hereinafter, exemplary embodiments of the present disclosure will be described in detail with reference to the accompanying drawings.

While the exemplary embodiments of the present disclosure are susceptible to various modifications and alternative forms, specific exemplary embodiments thereof are shown by way of example in the drawings and will herein be described in detail.

It should be understood, however, that the drawings and detailed description thereto are not intended to limit the exemplary embodiments of the present disclosure to the particular form disclosed, but on the contrary, the intention is to cover all modifications, equivalents, and alternatives falling within the spirit and scope of the present disclosure as defined by the appended claims.

The terms first, second, third and the like in the description and in the claims are used for distinguishing between similar elements and not necessarily for describing a sequential or chronological order. The terms are used only for distinguishing one component from another component.

In the specification, it is to be noted that the terms "comprising" or "including" and the like are not to be construed as necessarily including all of the several features, numbers, steps, operations, components or combinations thereof described in the specification. Rather, some of the several features, numbers, steps, operations, components or combinations thereof may not be included or additional several features, numbers, steps, operations, components or combinations thereof are construed as being further included. It is to be understood that when one element is referred to as being "connected to" or "coupled to" another element, it may be connected directly to or coupled directly to another element or be connected to or coupled to another element, having the other element intervening therebetween. In contrast, when an element is referred to as being "directly connected" or "directly coupled" to another element, there are no intervening elements present.

As used herein, an element referred by the term "module" or "unit" performs at least one function or operation. The module or unit performing the function or operation may be implemented as hardware, software, or a combination of hardware and software. In addition, a plurality of "modules" or a plurality of "units" other than those that are to be performed in a particular hardware or at least one processor may be integrated into at least one module. As used herein, the singular forms are intended to include plural forms as well, unless the context clearly indicates otherwise.

In other instances, well known methods, procedures, components, and circuits have not been described in detail as not to unnecessarily obscure aspects of the exemplary embodiments presented

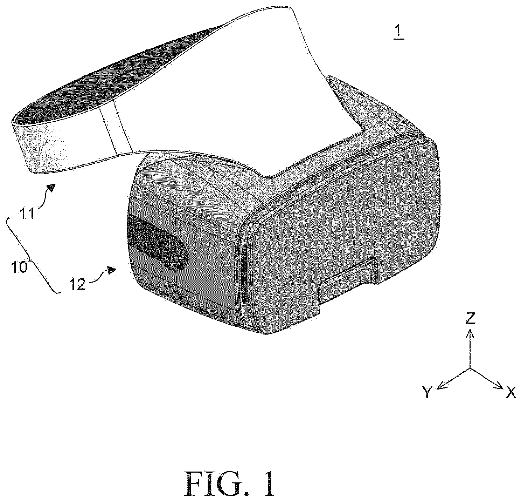

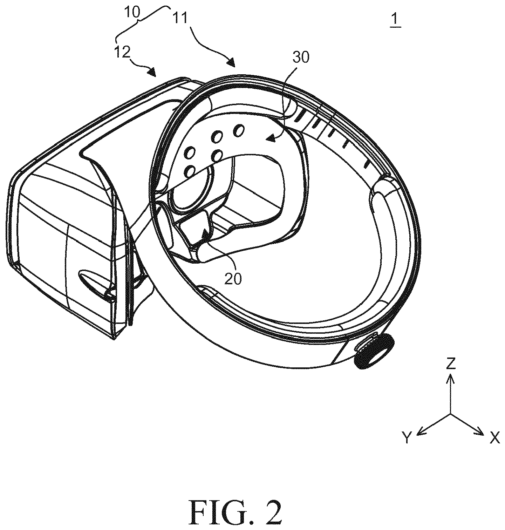

Referring to FIGS. 1 and 2, a head-mounted display device 1 according to an exemplary embodiment of the present disclosure (hereinafter referred to as a head-mounted display device 1) is worn on a user's head, provides the user with virtual reality (VR) images to allow for spatial and temporal experiences similar to the reality, and also measures the user's biometric data to sense physical, cognitive and emotional changes during the experience. The head-mounted display device 1 includes a display assembly 10.

The display assembly 10 can be worn on a user's head, and one side of the display assembly 10 is disposed to face the user's face so that the user can see a virtual reality image when the user wears the device. On the side of the display assembly 10, an image output means (not shown) for providing the user with a virtual reality image is disposed. For example, the image output means may include a portable terminal device such as a smart phone and a tablet PC, and a portable monitor connected to a PC to output an image provided from the PC.

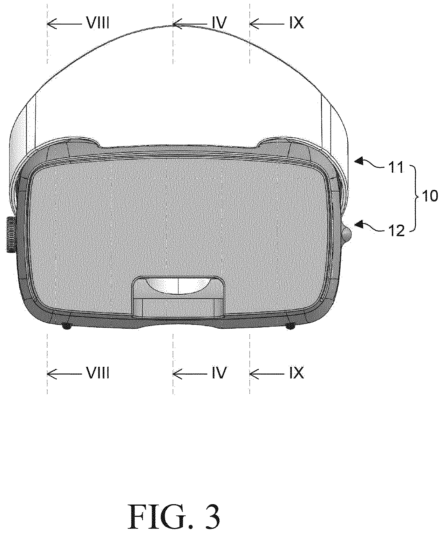

Referring to FIGS. 3 to 12, the display assembly 10 will be described in more detail.

The display assembly 10 may include a strap portion 11.

Referring to FIG. 3, the strap portion 11 may have in a structure that can be worn on the user's head. More specifically, the strap portion 11 may be formed in an annular band structure so as to surround a user's head when the user wears the device.

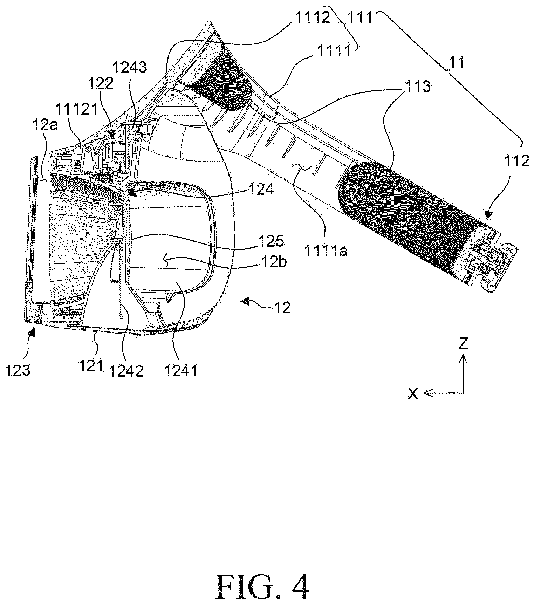

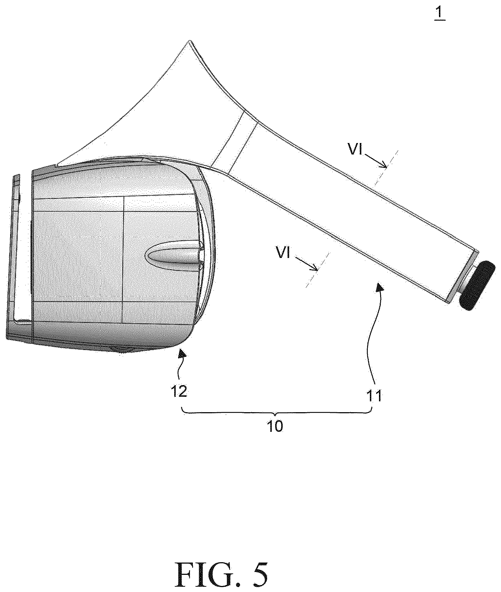

Referring to FIGS. 4 and 5, the strap portion 11 may include a strap body 111 and an inner-diameter adjusting means 112.

The strap body 111 may be formed in a circular or elliptical ring shape having a cut-away portion, such that it may include a head-accommodating space 1111a on its inner side in which a user's head is accommodated, and a band portion 1111 fitting around the user's head when the user wears the device. In addition, the strap body 111 may include a band support 1112 that is extended in a direction from one end of the band portion 1111 and rotatably coupled to a display body 12 to be described later. On one side of the band support 1112, a plurality of rotation supporting pieces 11121 may be formed, which protrudes outwardly from the inner side of the band support 1112 and accommodated in the display body 12 and is coupled with a rotation support portion 122 of the display body 12 to be described below. For example, the strap portion 11 may be made of a plastic material having a certain elasticity. A reinforcing means may be formed continuously along the inner surface of the strap portion 11 to supplement the strength of the strap portion 11.

The inner-diameter adjusting means 112 may be disposed at the cut-away portion of the band portion 1111 to connect both ends of the band portion 1111 separated from each other at the cut-away portion. The user may move the ends of the band portion 1111 to adjust the inner diameter of the strap body 111.

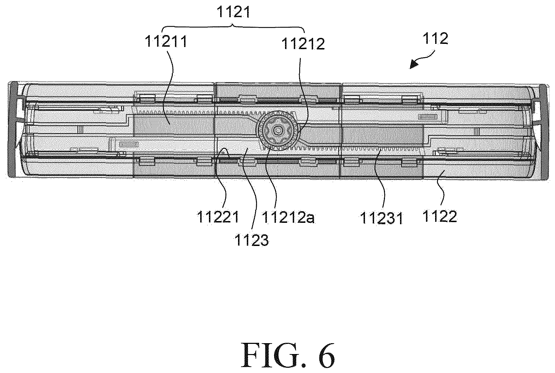

The inner-diameter adjusting means 112 will be described in more detail with reference to FIGS. 4 and 6.

The inner-diameter adjusting means 112 may include an adjustment portion 1121.

The adjustment portion 1121 may be disposed between the separated two ends of the band portion 1111, and may include a support plate 11211 that is coupled with the band portion 1111 to connect the separated two ends of the band portion 1111 to each other. For example, the support plate 11211 may be formed in an arc shape conforming to the inner surface of the band portion 1111 so as to be connected to the band portion 1111, and may have a fastening means such as a hook on one side thereof so as to be coupled with the band portion 1111. The adjustment portion 1121 may include a rotation adjusting member 11212 which is rotatably disposed at the central portion of the support plate 11211. The rotation adjusting member 11212 may be rotatably disposed at the center portion of the support plate 11211. The rotation adjusting member 11212 may include a grasping means disposed on the outer side of the band portion 1111 and a pinion gear 11212a disposed on the inner side of the band portion 1111.

Further, the inner-diameter adjusting means 112 may include a guide plate 1122.

The guide plate 1122 may be disposed on the inner surface of the band portion 1111 to be opposed to the support plate 11211 of the adjustment portion 1121 and may be formed in an arc shape conforming to the inner surface of the band portion 1111 so as to support the inner surface of the band portion 1111. A guide rail 11221 may be formed along the longitudinal direction of the guide plate 1122 so that a moving member 1123 to be described later may be disposed on a surface of the guide plate 1122 in contact with the inner surface of the band portion 1111. For example, more than one guide rails 11221 may be formed on the guide plate 1122 in the vertical direction of FIG. 6. In addition, the guide plate 1122 may have fastening means such as a hook on one side thereof to be coupled to the band portion 1111.

In addition, the inner-diameter adjusting means 112 may include the moving member 1123.

The moving member 1123 may be formed in an arc shape conforming to the inner surface of the guide plate 1122 so that it is accommodated in the guide rail 11221 and moves along the inner surface of the guide plate 1122. A rack gear 11231 engaged with the pinion gear 11212a of the rotation adjusting member 11212 may be formed on one side of the moving member 1123, and a coupling means coupled to the band portion 1111 may be formed on the other side of the moving member 1123. Therefore, when the rotation adjusting member 11212 is rotated, the moving member 1123 may move toward one side or the other side along the inner surface of the guide plate 1122 and may move the separated two ends of the band portion 1111 so that they become close to or away from the rotation adjusting member 11212, to adjust the inner diameter of the band portion 1111.

The strap portion 11 may further include a buffer portion 113.

Referring to FIG. 4, the buffer portion 113 may be disposed on the inner surface of the band portion 1111 and may support a user's head when the user wears the device. More specifically, the buffer portion 113 may include a mounting plate capable of being detachably attached to the inner surface of the band portion 1111, a cushioning material attached to one surface of the mounting plate, and an outer cover disposed on the outer surface of the cushioning material and in contact with the user's skin when the user wears the device. For example, the mounting plate may be formed in a shape conforming to the inner surface of the band portion 1111, and fastening means may be disposed on one surface of the mounting plate to fasten to the band portion 1111. The cushioning material may be formed of a material such as sponge and latex having a predetermined thickness. The outer cover may be formed of a material such as fabric, leather and nylon. More than one buffer portions 113 may be disposed on the inner surface of the band portion 1111. For example, the buffer portions 113 may be disposed at opposite positions, respectively, on the inner surface of the band portion 1111 so that they are located at front and rear of the user's head when the user wears the device.

The display assembly 10 may include a display body 12.

Referring to FIG. 4, the display body 12 is rotatably coupled to the strap portion 11, and may be positioned in front of a user's face as the user operates. A face-accommodating space 12b in which a part of a user's face is accommodated may be formed on one side of the display body 12, and a display-accommodating space 12a in which the image output means is accommodated may be formed on the other side of the display body 12.

The display body 12 will be described in more detail.

The display body 12 may include a housing portion 121.

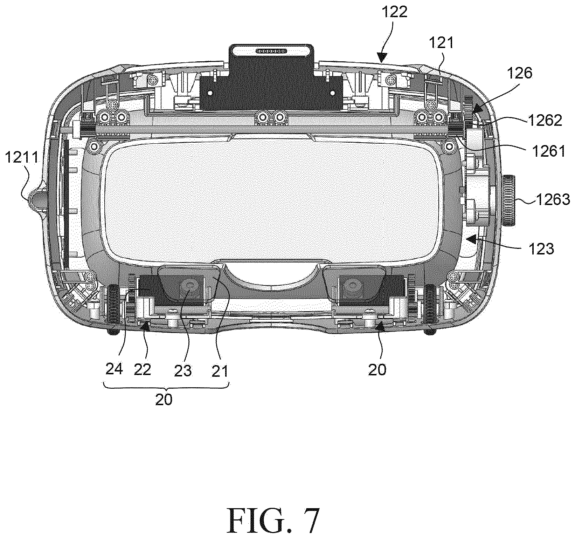

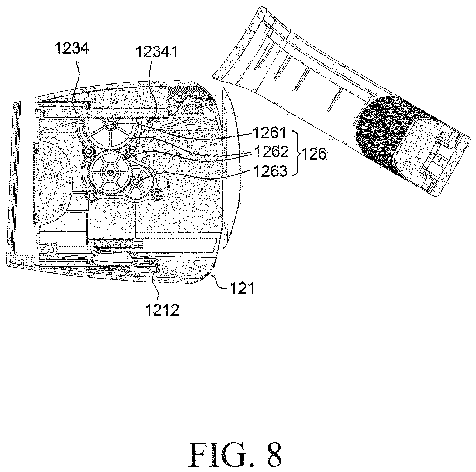

Referring to FIGS. 7 and 8, the housing portion 121 is formed in a tubular structure having openings at one end and the other end, respectively, which are connected to each other through the housing portion 121 in a direction. Accordingly, the housing portion 121 may accommodate therein the rotation support portion 122, a clip frame 123, a face-accommodating frame 124, a focusing means 126 and a first biometric data acquiring unit 20, which will be described in detail below. A cable protector 1211 may be formed on a side surface of the housing portion 121, which protrudes outwardly so as to surround and support a cable (not shown) connected to the display body 12. A through hole (not shown) may be formed in the other side surface of the housing portion 121 facing the cable protector 1211 so that a part of the focusing means 126 may be disposed therein. A movement restricting support 1212 may be formed on the inner surface of the housing portion 121, which guides the movement of the clip frame 123 and restricts the movement of the clip frame 123 to maintain the minimum spacing between the display-accommodating space 12a and lenses 125.

The display body 12 may further include the rotation support portion 122.

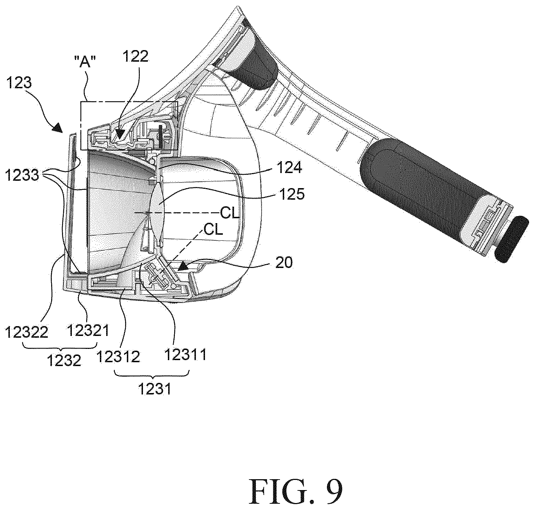

Referring to FIG. 9, the rotation support portion 122 may be coupled to the housing portion 121 and is accommodated in the upper side in the housing portion 121 and may be rotatably coupled with the rotation support piece 11121 of the strap portion 11. The rotation support portion 122 may limit the rotation angle of the display body 12.

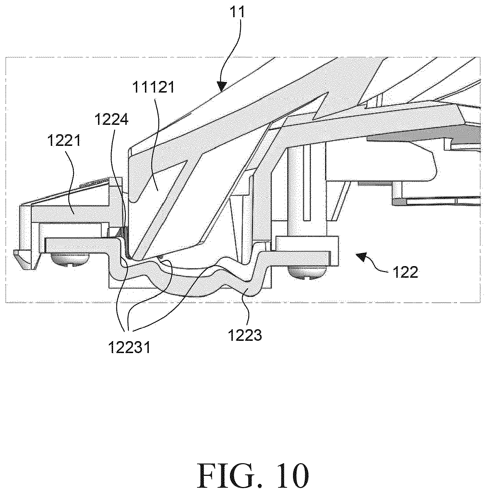

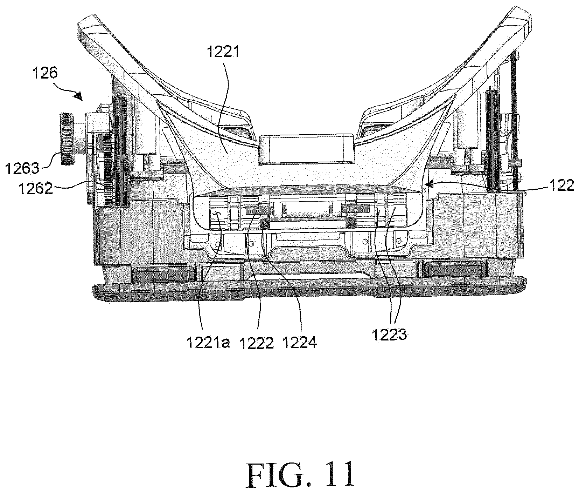

The rotation support portion 122 will be described in more detail with reference to FIGS. 10 and 11.

The rotation support portion 122 may include a rotation support body 1221, a rotation shaft 1222, a rotation restricting member 1223, and an elastic member 1224.

The rotation support body 1221 may be disposed in the housing portion 121 of the display body 12 and has an accommodating space therein so that a part of the strap portion 11, that is, the rotation support piece 11121 can be accommodated in it. For example, a support structure may be formed at the center portion of the rotation support body 1221, in which the rotation shaft 1222 is disposed. An accommodating space for accommodating the rotation support piece 11121 may be formed on either side of the center portion.

The rotation shaft 1222 is formed in the shape of a pin having a circular cross-section. As shown in FIG. 4, the rotation shaft 1222 may penetrate the rotation support body 1221 and a part of the strap portion 11 accommodated in the rotation support body 1221 (the rotation support piece 11121) to rotatably connect the rotation support body 1221 with the strap portion 11.

Referring back to FIGS. 10 and 11, the rotation restricting member 1223 is coupled with the lower surface of the rotation support body 1221. A plurality of projections 12231 may be formed on the surface of the rotation restricting member 1223 that faces a part of the strap portion 11 (the end of the rotation support piece 11121) so that a part of the strap portion 11 is stopped by the projections when the display body 12 is rotated. Accordingly, the user may rotate the display body 12 or the strap portion 11 upwardly or downwardly so that the end of the rotation support piece 11121 is stopped by one of the projections 12231, thereby adjusting the rotation angle of the display body 12 or the strap portion 11. For example, when the user wears the head-mounted display device 1, the user first rotates the strap portion 11 upwardly so that the rotation support piece 11121 is stopped and supported by the projections 12231, such that the strap portion 11 is fixed to the display body 12, tilted at a certain angle. Subsequently, while the strap portion 11 is tilted fixedly, the user brings the surface of the display body 12 into tight contact with her/his face, and then rotates the strap portion 11 downwardly to put the strap portion 11 on her/his head.

On the other hand, if the user puts on the strap portion 11 first, the user rotates the display body 12 upwardly so that the projections 12231 are stopped and supported by the rotation support piece 11121, such that the display body 12 is fixed to the strap portion 11, tilted at a certain angle. Subsequently, while the display body 12 is tilted fixedly, the user puts the strap portion 11 on her/his head, and then rotates the display body 12 downwardly to bring the surface of the display body 12 in tight contact with the user's face. That is to say, the rotation restricting member 1223 fixes either the display body 12 or the strap portion 11 depending on the order that the user wears the device so that they are tilted at a certain angle, thereby preventing the display body 12 or the strap portion 11 comes down to the user's face or head when the user puts it on. As a result, the user can easily and stably put on the device.

The elastic member 1224 may connect the rotation support body 1221 with a part of the strap portion 11 and may elastically support the rotation support body 1221. For example, the elastic member 1224 may be implemented as a tension coil spring having hooks at both ends thereof. More than one elastic members 1224 may be disposed on the inner side of the rotation support body 1221.

The display body 12 may further include the clip frame 123.

Referring to FIG. 9, the clip frame 123 is accommodated in the housing portion 121 to be disposed at one opening of the housing portion 121, and is connected to the focusing means 126, which will be described later. The clip frame 123 may be moved linearly toward one side or the other side along the direction in which the housing portion 121 is penetrated as being driven by the focusing means 126. The display-accommodating space 12a in which the image output means can be disposed may be formed on one side of the clip frame 123.

The clip frame 123 will be described in more detail.

The clip frame 123 may include a shielding portion 1231, a display support portion 1232, cushion pads 1233, and a linearly-moving member 1234.

The shielding portion 1231 is disposed around the front of the lenses 125 to be described later, and can block light or foreign substances from being introduced into the lenses 125. More specifically, the shielding portion 1231 may be accommodated in the housing portion 121 to be disposed in front of the face-accommodating frame 124, and may include a hood 12311 surrounding the front circumference of the lenses 125, and an extended portion 12312 extended outwardly from the end of the hood 12311 and supported on the inner surface of the housing portion 121.

The display support portion 1232 is disposed in front of the shielding portion 1231 to form the display-accommodating space 12a between the front surface of the shielding portion 1231 and the surface facing the front surface of the shielding portion 1231, and may support the image output means placed in the display-accommodating space 12a. More specifically, the display support portion 1232 may be disposed in front of the shielding portion 1231 and may include a first support portion 12321 for supporting a vertical load applied to the image output means, and a second support portion 12322 extended upwardly from the end of the first support portion 12321 for supporting the image output means supported by the first support portion 12321 toward the lenses 125. For example, the second support portion 12322 may be extended from the end of the first support portion 12321 such that it is inclined toward the lenses 125 at a predetermined angle. Although not shown in the drawings, an elastic plate (not shown) may be further disposed on the inner side of the display support portion 1232, which can bend the second support portion 12322 outwardly by a certain angle using an elastic force. By doing so, the width of the display-accommodating space 12a can be adjusted, so that image output means having different thicknesses can be mounted.

The cushion pads 1233 may be disposed on the front surface of the shielding portion 1231, on one surface of the first support portion 12321 exposed to the display-accommodating space 12a and on one surface of the second support portion 12322 facing the front surface of the shielding portion 1231, such that it may come in contact with the image output means (not shown).

For example, the cushion pads 1233 may absorb shock transmitted to the image output means and may be formed of a rubber or silicone material having a certain frictional force with respect to the object to support so as to prevent deviation of the image output means.

Referring to FIG. 8, the linearly-moving member 1234 may be disposed behind the shielding portion 1231 and may be extended in one direction by a predetermined length. A rack gear 12341 may be formed on one surface of the linearly-moving member 1234 along the longitudinal direction to be engaged with a pinion gear 1261 disposed at the focusing means 126. Accordingly, as the pinion gear 1261 of the focusing means 126 is rotated, the linearly-moving member 1234 may convert the rotational motion of the focusing means 126 into a linear motion to linearly move the shielding portion 1231.

The display body 12 may further include the face-accommodating frame 124.

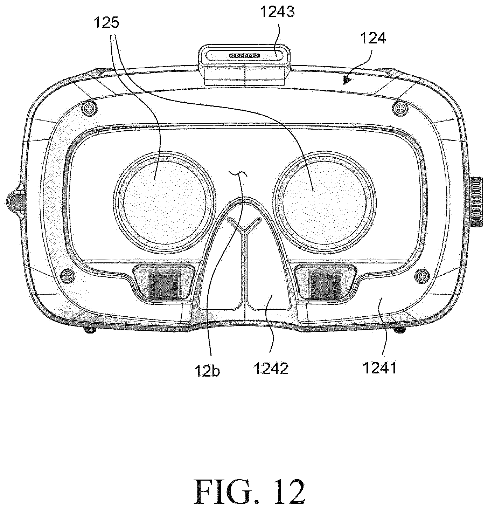

Referring to FIGS. 4 and 12, the face-accommodating frame 124 may be accommodated in the housing portion 121 to be disposed at the opening of the housing on the other side. The face-accommodating space 12b in which a part of the user's face is to be accommodated may be formed on the inner side of the face-accommodating frame 124.

The face-accommodating frame 124 will be described in more detail.

The face-accommodating frame 124 may include a face-accommodating body 1241, a shielding pad 1242, and a socket 1243.

The face-accommodating body 1241 may be disposed on the inner side of the housing portion 121 and may have a coupling means (not shown) on one surface thereof, which can be coupled with a second biometric data acquiring unit 30. For example, the coupling means may be implemented with velcro. It is, however, to be understood that the present disclosure is not limited thereto. The coupling means may be implemented in a variety of ways as long as it can perform the same functionality. The face-accommodating space 12b in which a part of the user's face is to be accommodated may be formed on the inner side of the face-accommodating frame 1241. Specifically, one side of the face-accommodating body 1241 in which the second biometric data acquiring unit 30 is to be disposed has a structure recessed to a predetermined depth toward the front, so that the face-accommodating space 12b in which the user's eyes and nose can be accommodated may be formed therein. When the user wears the head-mounted display device 1, the face-accommodating space 12b is shielded from light and remains dark, like a darkroom.

The shielding pad 1242 may be disposed at the face-accommodating body 1241 and disposed in the face-accommodating space 12b in which the user's nose is to be located. Therefore, when a user wears the head-mounted display device 1, the shielding pad 1242 is brought into tight contact with the user's nose located in the face-accommodating space 12b, such that the light introduced into the face-accommodating space 12b from below the display body 12. For example, the shielding pad 1242 may have a plurality of cut-out grooves formed therein and may be formed in a plate-like shape having a predetermined thickness. The shielding pad 1242 may be formed of rubber, silicone, a stretchable and flexible material, etc. so that the shielding pad 1242 having elasticity or stretchability can be in tight in contact with the user's nose when the shielding pad 1242 is pressed against the user's nose. In addition, a means for coupling to the face-accommodating body 1241 may be disposed on one surface of the shielding pad 1242.

The socket 1243 may be disposed at the face-accommodating body 1241 and electrically connected to a connector 33 disposed at the second biometric data acquiring unit 30. For example, the socket 1243 may include a connection pin connectable to a pin 332 of the connector 33, and a magnetic substance detachably attached to a magnet 333 of the connector 33.

The display body 12 may further include the lenses 125.

Referring to FIGS. 9 and 12, more than one lenses 125 may be disposed inside the face-accommodating frame 124 and may be arranged symmetrically with respect to the width direction of the face-accommodating frame 124. For example, annular fixing holes may be formed around the lenses 125 in the face-accommodating body 1241 to support the lenses 125. The lenses 125 may have a spherical structure having two convex surfaces.

The display body 12 may further include the focusing means 126.

Referring to FIGS. 7 and 8, the focusing means 126 may be accommodated in the housing portion 121 and coupled to the clip frame 123. The focusing means 126 may move the clip frame 123 according to the user's operation so that the display-accommodating space 12a can be spaced from the lenses 125 by a predetermined distance.

More specifically, the focusing means 126 may include the pinion gear 1261 engaged with the rack gear 12341 of the linearly-moving member 1234, and a power transmission gear 1262 for transmitting rotational power to the pinion gear 1261. The focusing means 126 may be rotatably disposed in the housing portion 121 so that one side thereof is coupled to the power transmission gear 1262 and the other side thereof is exposed out of the housing portion 121 to form a handle 1263 that the user can grasp. Accordingly, the user may turn the handle 1263 clockwise or counterclockwise to transmit the rotational power to the power transmission gear 1262. For example, more than one power transmission gears 1262 may be disposed. In addition, a gear protector for accommodating the power transmission gears 1262 therein may be disposed in the clip frame.

The head-mounted display device 1 further includes the first biometric data acquiring unit 20.

Referring to FIGS. 2 and 9, the first biometric data acquiring unit 20 is disposed on the inner, lower side of the display assembly 10, and captures the user's face accommodated in the display assembly 10 when the user wears the device to acquire the first biometric data from at least a part of the user's face. More specifically, the first biometric data acquiring unit 20 may acquire, from the captured image of the user's face accommodated in the display assembly 10, identification information based on the eyes' movement and the iris region around the pupil, changes in facial expressions based on muscular movements around the eyes, heart rate information based on changes in blood flow in the blood vessels around the eyes.

Referring to FIGS. 7 and 9, the first biometric data acquiring unit 20 may include an infrared ray (IR) filter 21 and an angle adjusting unit 22.

The IR filter 21 may be disposed at the face-accommodating frame 124 and may be inclined at a predetermined angle with respect to the central axis direction of the lenses 125. For example, the IR filter 21 may have a plate-like structure with a predetermined thickness and may be made of a transparent material that transmits infrared ray.

The angle adjusting unit 22 may be disposed rotatably within a predetermined angle between the clip frame 123 and the face-accommodating frame 124.

The angle adjusting unit 22 will be described in more detail.

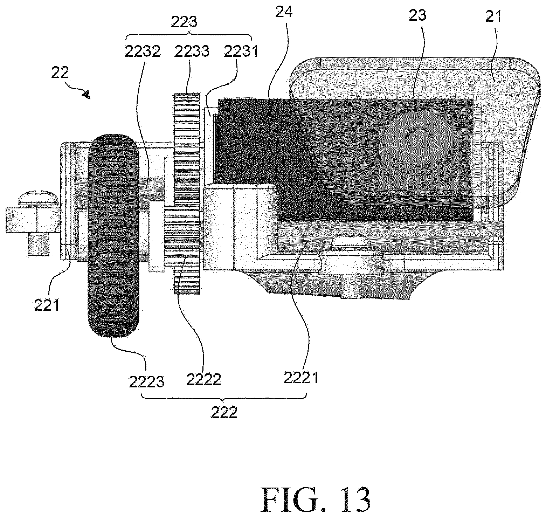

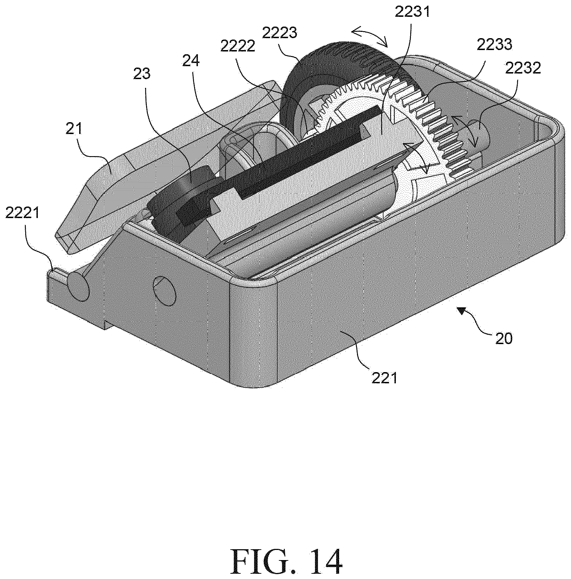

Referring to FIGS. 13 and 14, the angle adjusting unit 22 may include a frame 221, an operating part 222, and a rotating part 223.

The frame 221 may be disposed at the housing portion 121, and may have a space in which the operating part 222 and the rotating part 223 are accommodated. In addition, on either side of the frame 221, a through hole may be formed in which a main shaft 2221 of the operating part 222, and a driven shaft 2232 of the rotating part 223 are rotatably disposed, which will be described in detail below.

The operating part 222 is disposed at the frame 221 and is coupled to the rotating part 223 and can transmit rotational power to the rotating part 223 according to a user's operation. More specifically, the operating part 222 may include a main shaft 2221 rotatably coupled to the frame 221, a main gear 2222 coupled to the main shaft 2221 and rotatable with the main shaft 2221. In addition, the operating part 222 may include an operating means 2223 that is coupled to the main shaft 2221 to rotate with the main gear 2222 and the main shaft 2222. A part of the operating means 2223 is exposed out of the housing portion 121 so that the user can operate with it.

The rotating part 223 may be disposed at the frame 221 to be coupled with the operation part 222 and may receive the rotational power from the operation part 222 to be rotated clockwise or counterclockwise. More specifically, the rotation part 223 may include a rotating body 2231 accommodated in the frame 221. A capturing unit 23 and an IR light source 24, which will be described later, may be disposed on the inner side of the rotating body 223. The rotating body 223 may include the driven shaft 2232 coupled to the rotating body 2231 and rotatably coupled to the frame 221, and a driven gear 2232 coupled with one side of the driven shaft 2232 to be engaged with the main gear 2222 and rotated with the rotating body 2231 and the driven shaft 2232 as the main gear 2222 is rotated.

The first biometric data acquiring unit 20 may further include the capturing unit 23 and the IR light source 24.

Referring again to FIGS. 7 and 9, the capturing unit 23 is disposed at the angle adjusting unit 22 so that the angle can be adjusted according to the user's operation. In this manner, the capturing unit 23 may capture a particular portion or the whole of the user's face accommodated in the face-accommodating space 12b to acquire the first biometric data from at least a part of the user's face. For example, the capturing unit 23 may include a camera body including at least one lens therein, and an image sensor connected to the camera body. The capturing unit 23 located at the lower side of the display body 12 may be inclined at a predetermined angle with respect to the central axis of the lenses 125 so that the user's face accommodated in the face-accommodating space 12b can be captured. An infrared camera may be employed as the capturing unit 23.

The IR light source 24 is disposed around the capturing unit 23 and may emit infrared light that can pass through the IR filter 21 when the capturing unit 23 is driven. Further, the IR light source 24 may be disposed at the angle adjusting unit 22, so that the angle of the IR light source 24 may be adjusted according to a user's operation. Accordingly, the user may adjust the angle of the IR light source 24 to irradiate a particular portion or the whole of the user's face accommodated in the face-accommodating space 12b with light. For example, an infrared LED or an infrared lamp may be employed as the IR light source 24.

The head-mounted display device 1 further includes the second biometric data acquiring unit 30.

Referring to FIG. 2, the second biometric data acquiring unit 30 is detachably attached to the display assembly 10 and comes in contact with the user's skin when the user wears the device to acquire second biometric data, which is a biometric signal of the user.

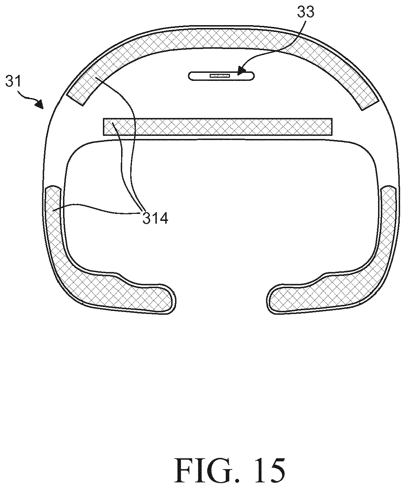

The second biometric data acquiring unit 30 will be described in more detail with reference to FIGS. 15 to 23.

Referring to FIGS. 2 and 15, the second biometric data acquiring unit 30 may include a mask body 31.

The mask body 31 may be formed in a curved structure along the width direction (y-axis direction) and the height direction (z-axis direction). The mask body 31 may have a viewing hole 311a formed therein which connects the lenses 125 disposed at the display assembly 10 with the user's eyes.

The mask body 31 may be detachably attached to the display assembly 10.

Referring to FIG. 15, coupling means 314 for fixing the mask body 31 to the display assembly 10 may be disposed on the surface of the mask body 31 that faces the display assembly 10. More specifically, a plurality of coupling means 314 may be disposed on the surface of the mask body 31 that is opposed to the surface of the mask body 31 where a sensing means 32 to be described later is disposed, and may be disposed at several regions along the edge of the mask body 31. For example, the coupling members 314 may be implemented with velcro.

It is, however, to be understood that the present disclosure is not limited thereto. The coupling means may be implemented in a variety of ways as long as they can perform the same functionality.

The mask body 31 may further include a frame 311.

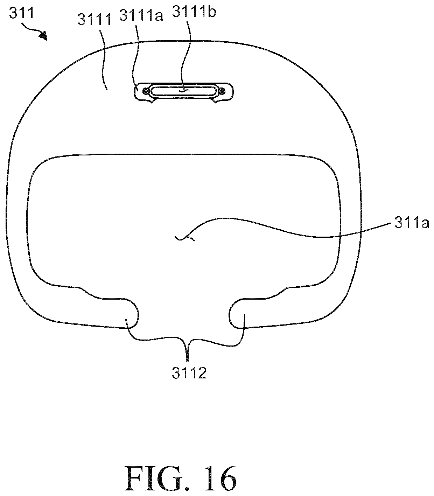

Referring to FIG. 16, the frame 311 may face and surround the user's forehead and the eyes when the user wears the device. In addition, a viewing hole 311a may be formed on the inner side of the frame 311, in which the user's eyes and nose may be located.

The frame 311 will be described in more detail.

The frame 311 may include a first support portion 3111 and a second support portion 3112.

The first support portion 3111 may face the user's forehead when the user wears the device. A mounting groove 3111a may be formed at the center portion on the inner side of the first support portion 3111, which is depressed to a certain depth in a direction (x-axis direction in FIG. 1) so that the connector 33 to be described later may be disposed. In the mounting groove 3111a, a through hole 3111b may be formed so that a part of the connector 33 disposed at the mounting groove 3111 a is exposed to the outside of the frame 311. For example, in the mounting groove 3111a, a fastening hole may be further formed, in which a means for fastening the connector 33 to the first support portion 3111 (not shown) can be fastened.

The second support portion 3112 may be extended from one end and the other end of the first support portion 3111 and bent in the width direction of the mask body 31 (the y-axis direction in FIG. 2) at the lower portion of the mask body 31. In other words, the second support portion 3112 may be extended downwardly from the ends of the mask body 31 in the height direction of the mask body 31 (the z-axis direction in FIG. 2) into curves. In addition, the second support portion 3112 may include a first extension portion extended in the vertical direction from the ends of the first support portion 3111 and a second extension portion extended in the horizontal direction from the ends of the first extension portion so that the second support portion 3112 may face the user's side head and zygoma when the user wear it. Accordingly, the viewing hole 311a may be formed on the inner side of the first support portion 3111 and the second support portion 3112, in which the user's eyes and nose may be accommodated. Although not shown in the drawings, the second support portion 3112 may have a structure that allows the length thereof to be adjusted along the height direction of the mask body 31 (the z-axis direction in FIG. 2). At this time, a length adjusting member (not shown) for guiding the movement of the second support portion 3112 and fixing the second support portion 3112 after it is moved to a predetermined position may be disposed on the inner side of the first support portion 3111 and the second support portion 3112. Accordingly, the user can adjust the length of the second support portion 3112 appropriately for the positions of her/his eyes and nose to thereby stably support the display assembly 10.

For example, the frame 311 may be made of a plastic material or a carbon material which has a certain strength against bending. The frame 311 may include a plurality of through holes passing through the frame 311 in order to make the frame portion 311 lighter. It is, however, to be understood that the material of the frame 311 is not limited thereto.

The mask body 31 may further include a buffer portion 312.

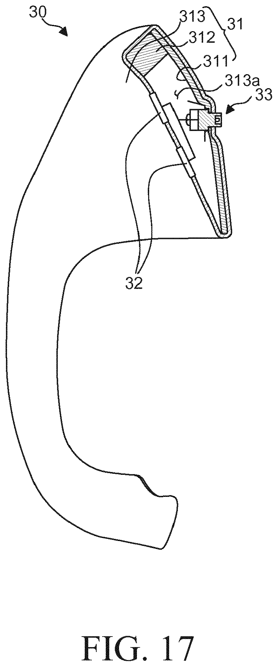

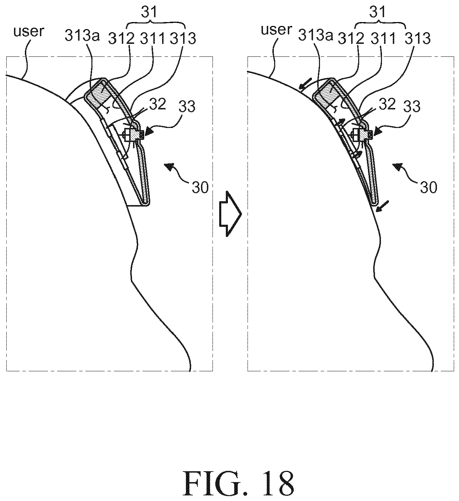

Referring to FIGS. 17 and 18, the buffer portion 312 may be disposed on one surface of the frame 311 facing the user's face and may support the user's face when the user wears the device so that the load of the display assembly 10 may be evenly distributed on the user's face.

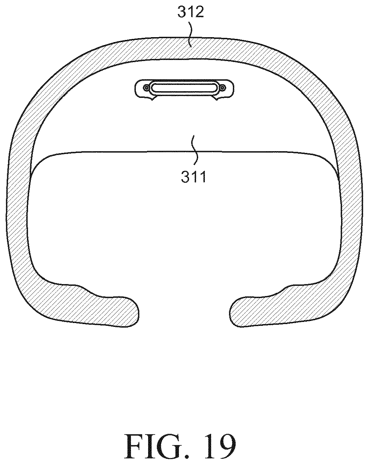

For example, the buffer portion 312 may be implemented as a foam made of a PE material having a predetermined thickness. It is, however, to be understood that the material of the buffer portion 312 is not limited thereto. The buffer portion 312 may be made of other materials such as a sponge, a gel, a latex and an air foam as long as it can perform the same function. As shown in FIG. 19, the buffer portion 312 may be disposed on a surface of the frame 311 and may be disposed along the edge of the frame 311.

The mask body 31 may further include an elastic member 313.

Referring to FIGS. 17 and 18, the elastic member 313 may be made up of an elastic fabric material having elasticity to surround the frame 311 and the buffer portion 312 and may support the sensing means 32 to be described below. A buffer space 313a may be formed between the frame 311 and the buffer portion 312 disposed at the edge of the frame 311. When the user puts on the device, the elastic member 313 is pressed against the user's forehead and is pushed toward the buffer space 313a while being stretched outwardly, such that it is elastically deformed into a shape conforming to the user's forehead. As a result, the sensing means 32 disposed inside the elastic member 313 can be elastically supported so that the sensing means 32 can be in tight contact with the user's forehead. For example, the elastic member 313 may be made of a material having a higher elastic modulus than that of the buffer portion 312. The surface of the elastic member 313 may be formed of nylon, suede, etc. so as to reduce the frictional force when it comes in contact with the user's skin.

The second biometric data acquiring unit 30 may include the sensing means 32.

Referring to FIGS. 17 and 18, the sensing means 32 is disposed on the of the mask body 31 that faces the user's face when the user wears the device, and is elastically supported by the elastic member 313 of the mask body 31 to be in contact with the user's skin. Accordingly, the sensing means 32 can sense the second biometric data, which is a biometric signal from the user. In addition, the sensing means 32 may be implemented as a disk-shaped electrode made of a metal material or a thin-film electrode printed on a flexible substrate, and may sense the biometric signal from the user when it is connected to the display assembly 10 and receives power. For example, the sensing means 32 may be disposed on one side of the mask body 31 by pressing or bonding it. The sensing means 32 may be further subjected to a process for EMI shielding (Electro Magnetic Interference Shield) for shielding electromagnetic waves.

That is to say, the head-mounted display device 1 may include the buffer portion 312 which is disposed on one side of the mask body 31 to support a user's face so that the load of the display assembly 10 is evenly distributed on the user's face, and an elastic member 313 which is formed of an elastic fabric material and is stretched outwardly when it is pressed against the user's forehead so that it comes in tight contact with the user's forehead. In this manner, the sensing means 32 can be in contact with the user's skin stably and reliably. In addition, the display assembly 10 can stably support the user's face, and thus it is possible to prevent the display assembly 10 from being deviated from the user's face as the user takes actions. In addition, as the sensing means 32 is in tight contact with the user's forehead, it is possible prevent a gap from being formed between the user's skin and the sensing means 32, thereby preventing the sensing means 32 from being separated. In addition, the sensing means 32 is elastically supported to thereby reduce the pressure applied to the user's forehead, such that it is possible to avoid a mark or a pain on the user's skin.

The sensing means 32 may have a structure that can be brought into tight contact with the user's forehead by using its own elastic force.

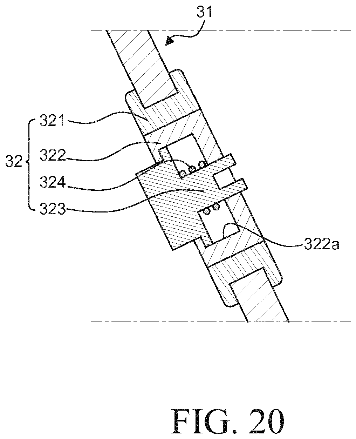

Referring to FIG. 20, the sensing means 32 may be disposed at the mask body 31 and may have an elastically-supported structure so that it is moved linearly in one direction when an external force is applied from the outside to be compressed, and is moved linearly in the opposite direction to be restored to its original state by the elastic force when the external force is released.

The sensing means 32 having the elastically-supported structure will be described in more detail.

The sensing means 32 may include a support ring 321, a guide housing 322, an electrode 323 and an elastic support member 324. The support ring 321 may be formed in a annular shape with a press-fit groove that is recessed toward the inner center and is disposed along the circumference, and may be disposed at the mask body 31. For example, the support ring 321 may be disposed at the mask body 31 by pressing it. The guide housing 322 may be fastened to the inside of the support ring 321, and a guide hole 322a may be formed along the center axis direction therein. A step for restricting the movement of the electrode 323 may be formed in the guide housing 322. For example, threads that can be engaged with each other may be formed on the outer circumferential surface of the guide housing 322 and the inner circumferential surface of the support ring 321, respectively. The electrode 323 may be disposed inside the guide housing 322 and linearly movable along the guide hole 322a. The side and the other side of the electrode 323 may protrude out of the guide housing 322, respectively. A protruding piece may be formed on one side of the electrode 323, which is held by the step formed in the guide housing 322. The elastic support member 324 may be disposed inside the guide housing 322 and may be in the form of a coil spring to elastically support the electrode 323.

The sensing means 32 having the elastically-supported structure may be disposed at the elastic member 313 of the mask body 31 or at the buffer portion 312 disposed on the surface of the frame 311. If the sensing means 32 is disposed at the buffer portion 312, the buffer portion 312 may be formed in a shape conforming to the surface of the frame 311 so as to be in contact with the user's skin.

At this time, the elastic member 313 may not be disposed at the mask body 31.

The sensing means 32 may face a user's frontal lobe region when the user wears the device to sense the user's brain wave signal.

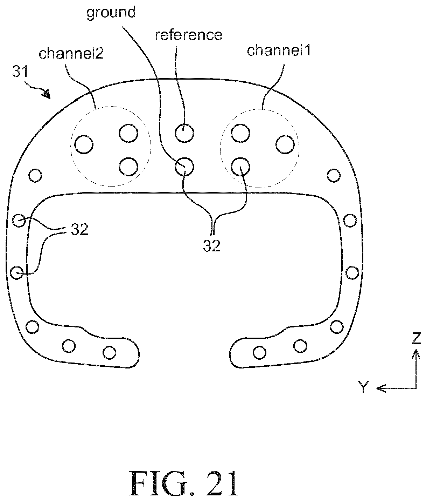

Referring to FIGS. 18 and 21, the sensing means 32 may be disposed at positions where the user's forehead is located when the user wears the device. Specifically, the sensing means may be disposed at the positions that face the surface of the first support portion 3111 where the user's forehead is located when the user wears the device. More than one sensing means 32 may be disposed. More specifically, the sensing means 32 may include a ground, a reference, and measurement terminals (Channel 1 and Channel 2). For example, the ground and the reference may be disposed at positions corresponding to the center of the first support 3111 and may be spaced apart from each other by a predetermined distance in the height direction of the mask body 31 (the z-axis direction). The measurement terminals Channel 1 and Channel 2 may be disposed on one side and the other side of the first support portion 3111 in the width direction of the mask body 31 (the y-axis direction) such that they are symmetrically with respect to the center portion of the first support portion 3111 where the ground and the reference are located. For example, more than one measurement terminals (Channel 1 and Channel 2) may be arranged.

In addition, the sensing means 32 may be disposed at the position where the user's side head and zygoma are located when the user wears the device, so that the user's biological signal can be sensed.

Referring to FIGS. 16 and 21, the sensing means 32 may be disposed at positions that face the surface of the second support portion 3112 such that it faces the user's side head and zygoma when the user wears the device. The sensing means 32 in contact with the user's side head and zygoma may sense at least one of the user's photoplethysmogram (PPG), electrocardiogram (ECG), electromyography (EMG) and skin temperature.

Although not shown in the drawings, the sensing means 32 may be electrically connected to other sensing means in contact with a user's body part other than the face. Accordingly, biometric signals other than the above-described biometric signal can be additionally sensed. For example, the sensing means 32 connected to the other sensing means may additionally sense body fat (a bioelectrical impedance analysis or BIA), stress response (a galvanic skin response or GSR), etc.

The sensing means 32 may have a structure that allows its position to be adjusted inside the mask body 31.

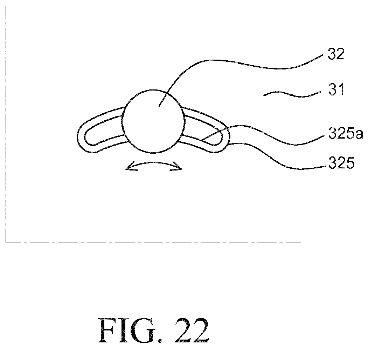

Referring to FIG. 22, a movement adjusting member 325 for guiding the movement of the sensing means 32 according to a user's operation may be disposed on the mask body 31. The movement adjusting member 325 may be formed in a ring shape, and a guide hole 325a for guiding the movement of the sensing means 32 may be formed in the movement adjusting member 325. Although the guide hole 325a is shown as an arc shape curved along the horizontal direction in FIG. 22, the shape of the guide hole 325a is not limited thereto. The shape of the guide hole 325a may be altered into a variety of shapes as long as it can guide the movement of the sensing means 32 in the horizontal or vertical direction.

The second biometric data acquiring unit 30 may further include the connector 33.

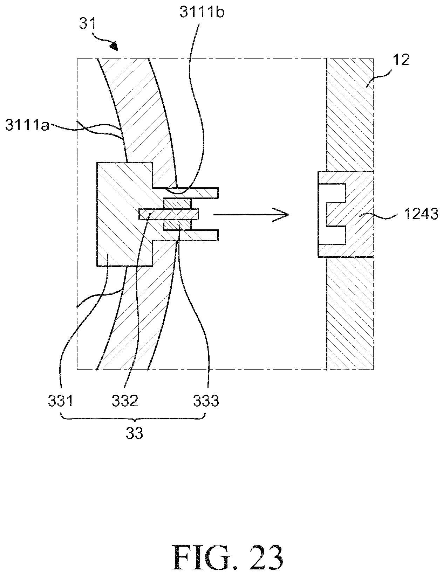

Referring to FIGS. 17 and 23, the connector 33 may be disposed at the mask body 31 to be electrically connected to the sensing means 32, and may be connected to the socket 1243 formed in the display assembly 10 to transmit at least one second biometric data sensed by the sensing means 32 to a control means (not shown). The connector 33 may be coupled to the socket 1243 of the display assembly 10 using a magnetic force. For example, the connector 33 may include a connector housing 331 disposed at the mask body 31, a pin 332 electrically connected to the sensing means 32 and disposed at an inner central portion of the connector housing 331, and a magnet 333 disposed around the pin 332. The connector 33 can be coupled to the socket 1243 by the magnetic force only when the connector 33 is aligned with the center of the socket 1243. Accordingly, the user can quickly connect the connector 30 with the socket. The connector 33 can be more stably fixed to the socket 1243 by the magnetic force.

As described above, according to the exemplary embodiment of the present disclosure, the head-mounted display device can not only provide a user with virtual reality image but also acquire biometric data from the user's face who is watching the virtual reality image to thereby check the user's cognitive and emotional conditions. Accordingly, it is possible to develop contents including various experience elements. Furthermore, it is possible to improve satisfaction of the user's experience by providing contents suitable for the user's current conditions in real-time.

Further, since the display body 12 is rotatably coupled to the strap portion 11 and the strap portion 11 is in the form of an annular structure which can be put on by one hand, the user can easily put it on and take it off to look around as desired. As a result, the convenience can be improved.

Further, the display body 12 can be stably fixed to the user's face by the annular strap portion 11 in the form of the structure. Furthermore, even if the user takes a strenuous action during the experience, it is possible to prevent the display body 12 from being deviated from the user's face.

Further, as the mask body 31 supporting the user's face can be detached from the display body 12, it is possible to easily replace the mask body 31, thereby increasing the lifetime of the device while saving the cost.

Although the preferred embodiments of the present invention have been disclosed for illustrative purposes, those skilled in the art will appreciate that various modifications, additions and substitutions are possible, without departing from the scope and spirit of the invention as disclosed in the accompanying claims. Accordingly, such modifications, additions and substitutions should also be understood to fall within the scope of the present invention.

### [INDUSTRIAL APPLICABILITY]

The head-mounted display device according to the exemplary embodiment of the present disclosure can be used in industrial fields such as games, education, national defense, medical care, etc., in which spatial and temporal experiences similar to the reality can be realized.

## Claims

1. A head-mounted display device comprising:
a display assembly disposed to face a user's face when the user wears the device and configured to provide the user with virtual reality images via an image output means disposed on one side thereof;
a first biometric data acquiring unit to acquire first biometric data, disposed at the display assembly and configured to capture the user's face accommodated in the display assembly when the user wears the device; and
a second biometric data acquiring unit to acquire second biometric data, disposed at the display assembly and in contact with the user's skin when the user wears the device.

2. The device of claim 1, wherein the display assembly comprises:
a strap portion wearable on the user's head; and
a display body rotatably coupled to the strap portion and positioned in front of the user's face as the user operates, wherein the display body has a face-accommodating space on one side thereof where the user's face is to be accommodated, and a display-accommodating space on an opposite side thereof where the image output means is to be accommodated.

3. The device of claim 2, wherein the strap portion comprises:
a strap body comprising a band portion having a head-accommodating space formed therein for receiving the user's head and fitting around the user's head when the user wears the device, and a band support extended from the band portion and rotatably coupled to a rotation support portion;
an inner-diameter adjusting means disposed at the strap body to allow the user to adjust an inner diameter of the strap body; and
a buffer portion disposed on an inner surface of the band portion to support the user's head when the user wears the device.

4. The device of claim 3, wherein the inner-diameter adjusting means comprises:
an adjustment portion comprising a support plate coupled to the band portion, and a rotation adjusting member rotatably disposed at a center of the support plate and having a pinion gear on one side thereof;
a guide plate disposed on an inner surface of the band portion to face the adjustment portion and has a guide rail formed on one surface thereof in a longitudinal direction; and
a moving member accommodated in the guide rail and having a rack gear on one side thereof to be engaged with the pinion gear, and a coupling means coupled to the band portion on an opposite side thereof, wherein the moving member moves toward one side or an opposite side when the rotation adjusting member is rotated to adjust the inner diameter of the band portion.

5. The device of claim 2, wherein the display body comprises:
a housing portion having openings at one end and an opposite end thereof, the openings being connected to each other through the housing portion in a direction;
a rotation support portion accommodated in the housing to be rotatably coupled to the strap portion and restricting an rotation angle of the display body;
a clip frame disposed at the opening at the one end of the housing, having the display-accommodating space on one side thereof, and being linearly movable through the housing portion in the direction;
a face-accommodating frame disposed at the opening at the opposite end of the housing portion and having the face-accommodating space therein;
lenses disposed in the face-accommodating frame; and
a focusing means coupled to the clip frame and configured to move the clip frame according to the user's operation to separate the display-accommodating space from the lenses by a predetermined display.

6. The device of claim 5, wherein the rotation support member comprises:
a rotation support body disposed at the display body and capable of accommodating a part of the strap portion;
a rotation shaft penetrating the rotation support body and a part of the strap portion accommodated in the rotation support body to rotatably connect the rotation support body with the strap portion;
a rotation restricting member coupled to the rotation support body and having projections on a surface thereof that faces the part of the strap portion so that the part of the strap portion is held by the projections when the display body is rotated; and
an elastic member connecting the rotation support body with the part of the strap portion and elastically support the rotation support body.

7. The device of claim 5, wherein the clip frame comprises:
a shielding portion accommodated in the housing portion to be disposed in front of the face-accommodating frame and comprising a hood surrounding the lenses and an extended portion extended from an end of the hood outwardly to be supported on an inner surface of the housing portion;
a display support portion disposed in front of the shielding portion and comprising a first support portion for supporting a vertical load applied to the image output means, and a second support portion extended upwardly from an end of the first support portion for supporting the image output means supported by the first support portion toward the lenses;
a cushion pad disposed at the first support portion and the second support portion to be in contact with the image output means; and
a linearly-moving member disposed at the shielding portion, having a rack gear formed on a surface thereof in a longitudinal direction to be coupled with the focusing means, and converting a rotation movement of the focusing means into a linear movement to move the shielding portion linearly.

8. The device of claim 5, wherein the face-accommodating frame comprises a face-accommodating body having a coupling means to be coupled with the second biometric data acquiring unit on one surface thereof and having the face-accommodating space where the user's eyes and nose are to be accommodated;
a shielding pad disposed at the face-accommodating body and in tight contact with the user's nose when the user wears the device to block light from being introduced into the face-accommodating space; and
a socket disposed at the face-accommodating body and electrically connected to the second biometric data acquiring unit.

9. The device of claim 7, wherein the focusing means comprises:
a pinion gear engaged with the rack gear of the linearly-moving member;
a power transmission gear for transmitting rotational power to the pinion gear; and
a handle coupled to the power transmission gear, partially exposed out of the housing portion, and transmitting rotational power to the power transmission gear according to the user's operation.

10. The device of claim 5, wherein the first biometric data acquiring unit comprises:
an IR filter disposed in the face-accommodating frame and inclined with respect to a center axis of the lenses at a predetermined angle;
an angle adjusting unit disposed between the clip frame and the face-accommodating frame and rotatable within a predetermined angle;
a capturing unit disposed at the angle adjusting unit and capable of capturing at least a part of the user's face to acquire the first biometric data; and
an IR light source disposed at the angle adjusting unit and disposed around the capturing unit to emit light.

11. The device of claim 10, wherein the angle adjusting unit comprises:
a frame disposed in the housing portion and having an accommodating space therein;
an operating part comprising a main shaft rotatably coupled to the frame, a main gear coupled to the main shaft and rotatable with the main shaft, and an operating means coupled to the main shaft to rotate with the main gear and the main shaft, wherein a part of the operating means is exposed out of the housing portion so that the user can operate with it; and
a rotating body comprising a rotating body accommodated in the frame, wherein the capturing unit and the IR light source are disposed on an inner side thereof, a driven shaft coupled to the rotating body and rotatably coupled to the frame, and a driven gear coupled with one side of the driven shaft to be engaged with the main gear and rotated with the rotating body and the driven shaft as the main gear is rotated.

12. The device of claim 1, wherein the second biometric data acquiring unit comprises:
a mask body having a coupling means on one surface thereof that faces the display assembly, and being detachably attached to the display assembly;
a sensing means disposed at the mask body and coming in contact with the user's skin when the user wear the device to sensing a biometric signal from the user to acquire the second biometric data; and
a connector disposed at the mask body and electrically connected to the sensing means, wherein the connector is connected to the socket disposed at the display assembly to transmit the second biometric data sensed by the sensing means.
